Europäisches Patentamt

European Patent Office

Office européen des brevets

(19)

(11) Veröffentlichungsnummer: **0 234 207**
**A2**

(12) # EUROPÄISCHE PATENTANMELDUNG

(21) Anmeldenummer: 87100241.6

(51) Int. Cl.4: **A61K 31/70**

(22) Anmeldetag: 10.01.87

(30) Priorität: 29.01.86 DE 3602670

(43) Veröffentlichungstag der Anmeldung:
02.09.87 Patentblatt 87/36

(84) Benannte Vertragsstaaten:
AT BE CH DE ES FR GB GR IT LI LU NL SE

(71) Anmelder: **Speck, Ulrich, Dr.**
**Benediktinerstrasse 50**
**D-1000 Berlin 28(DE)**

(72) Erfinder: **Speck, Ulrich, Dr.**
**Benediktinerstrasse 50**
**D-1000 Berlin 28(DE)**

(74) Vertreter: **Wablat, Wolfgang, Dr.**
**WABLAT & PARTNER Potsdamer Chaussee 47**
**D-1000 Berlin 38(DE)**

(54) Verwendung von N-Acetylglucosamin zur Therapie degenerativer Gelenkprozesse und verwandter Erkrankungen.

(57) Die Erfindung betrifft die Verwendung von N-Acetylglucosamin zur Therapie degenerativer Erkrankungen der Gelenke und des Binde-und Stützgewebes sowie verwandter Erkrankungen. Ferner umfaßt die Erfindung die Verwendung von N-Acetylglucosamin zur Herstellung von Arzneimitteln für die entsprechende Therapie.

EP 0 234 207 A2

## Verwendung von N-Acetylglucosamin zur Therapie degenerativer Gelenkprozesse und verwandter Erkrankungen

Die Erfindung betrifft die Verwendung von N-Acetylglucosamin für die Therapie degenerativer Erkrankungen der Gelenke und des Binde-und Stützgewebes sowie verwandter Erkrankungen. Ferner umfaßt die Erfindung die Verwendung von N-Acetylglucosamin zur Herstellung von Arzneimitteln für die entsprechende Therapie.

Degenerative Erkrankungen der Gelenke sind bei Menschen und Tieren mittleren und höheren Alters weit verbreitet. Sie sind eine wesentliche Ursache für schlechtes Allgemeinbefinden und eingeschränkte Arbeitsfähigkeit. Eine Hauptursache für die Erkrankung ist ein Abbau der Glycosaminoglycane (GAG), die ein wesentlicher Bestandteil der Knorpelsubstanzen und anderer elastischer Elemente sind sowie zur Gleitfähigkeit der Gelenke beitragen.

Die Therapie der schmerzhaften, teils auch entzündlichen Zustände erfolgt häufig nur symptomatisch mit Hilfe von nichtsteroidalen Entzündungshemmern, wie beispielsweise Indo methacin oder sogar durch Einsatz von Kortikoiden. Beide Gruppen von Therapeutika verursachen gravierende Nebenwirkungen und sollten daher so wenig wie möglich eingesetzt werden. Darüber hinaus besteht bei der Anwendung der nicht-steroidalen Entzündungshemmer und bei den Kortikoiden die Gefahr einer weiteren Verschiebung des Stoffwechsels der GAGs in Richtung auf einen beschleunigten Abbau. Dem Vorteil der momentanen Linderung der Symptome der Erkrankung, wie Schmerz und Unbeweglichkeit der Gelenke, steht daher neben anderen Risiken die Gefahr einer Beschleunigung der degenerativen Prozesse, welche die Krankheit verursachen, gegenüber.

Seit langem ist bekannt, daß demgegenüber Glycosaminoglycane oder auch die Vorstufe eines Bausteins der GAGs, das Glucosamin, eine ursächliche therapeutische Wirkung ausüben. Die Wirkung beruht einerseits auf einem Einbau der betreffenden Bausteine in die GAGs, andererseits in einer Stimulierung der Neusynthese von GAGs durch eine Erhöhung der Konzentration von Vorstufen ihrer Synthese. Auch eine Hemmung de enzymatischen Abbauprozesse wird angenommen. Damit besteht die Möglichkeit, die für die Erkrankung ursächlichen Stoffwechselprozesse günstig zu beeinflussen und damit zu einer Heilung oder zumindestens Verlangsamung der degenerativen Vorgänge, welche der Erkranung zugrunde liegen, beizutragen.

Nun sind allerdings die für die zuletzt erwähnte ursächliche Therapie zur Verfügung stehenden Arzneistoffe ebenfalls noch nicht ideal.

Aus biologischem Material isolierte GAGs weisen den Nachteil komplexer Naturprodukte auf: sie sind nur schwer oder kaum eindeutig zu definieren; ihre parenterale Anwendung ist notwendig, um eine ausreichende Bioverfügbarkeit zu gewährleisten. Daneben besteht immer die Gefahr anaphylaktischer Reaktionen. Die begrenzte Löslichkeit und die hohe Viskosität konzentrierter Lösungen erschweren die Verabreichung in der wünschenswert hohen Dosierung.

Anstelle der natürlichen GAGs wurde mit gutem therapeutischen Erfolg auch Glucosaminsulfat oral, intramuskulär und intraartikulär verabreicht. Glucosaminsulfat hat den großen Vorteil, eine im Hinblick auf Identität, Reinheit und Stabilität eindeutig definierbare Verbindung zu sein. Glucosaminsulfat verursacht als niedermolekulare, natürliche Substanz keine Allergien und läßt in der notwendigen Dosierung kaum toxische Wirkungen erwarten. Andererseits weist auch Glucosaminsulfat wesentliche Nachteile auf, wie sie sich beispielsweise aus der Basisinformation Dona®200-S der Firma Opfermann-Arzneimittel, 5060 Bergisch Gladbach 2, ablesen lassen:

Die orale Applikationsform ist offenbar sehr viel weniger wirksam als die intravenöse oder intramuskuläre Injektion. Es wird eine orale Wochendosis von 5250 mg empfohlen, wogegen parenteral nur 1200 mg notwendig sind. Das wirksamere Injektionspräparat ist in Lösung bei physiologischem pH-Wert nicht ausreichend stabil; es wird daher bei saurem pH-Wert zubereitet, gelagert und geliefert und muß vor Gebrauch vom Arzt neutralisiert werden. Zu diesem Zweck wird der Glucosaminsulfatlösung eine Pufferlösung zugesetzt. Glucosaminsulfatlösung und Puffer haben insgesamt bei der notwendigen hohen Dosierung und Konzentration einen gegenüber dem Blut so hohen osmotischen Druck, daß zusätzlich Lidocain als Lokalanästhetikum zugesetzt werden muß. Erst durch diesen Zusatz wird die Injektion in die Gelenke ausreichend schmerzarm. Damit ist das an sich untoxische Glucosaminsulfat mit den Nebenwirkungen des Lidocains (Herz-Kreislaufreaktionen, Schwindel, Erbrechen) belastet.

Andere Präparationen, wie sie in der erst kürzlich publizierten Anmeldung EP 85 112 913.0 beschrieben werden, beinhalten komplexe Gemische von Einzelkomponenten, die eine Reihe von offensichtlichen Nachteilen aufweisen:

a) Die chemische Stabilität jedes der Bestandteile in dem Gemisch ist häufig nur schwer erreichbar, insbesondere unter den Bedingungen der Langzeitlagerung.

b) Die Gesundheitsbehörden vieler Länder verhalten sich aus guten Gründen bei der Zulassung von Kombinationspräparaten sehr restriktiv.

So wird der Nachweis verlangt, daß jeder Bestandteil wirksam ist, zusätzlich häufig, daß die feste Kombination der Einzelkomponenten spezifische Vorteile bietet. Beide Beweise sind für Kombinatinen von mehr als zwei Wirkstoffen kaum mehr zu erbringen.

Aufgabe der Erfindung ist es somit, die zuvor beschriebenen Nachteile zu überwinden und ein Mittel mit lediglich einem Wirkstoff zur Verwendung in der Therapie degenerativer Erkrankungen der Gelenke und des Binde-und Stützgewebes sowie verwandter Erkrankungen zur Verfügung zu stellen, der für sich allein verabreicht das Fortschreiten der Erkrankung verzögert, verhindert oder Heilungsprozesse in Gang setzt.

Erfindungsgemäß wird diese Aufgabe dadurch gelöst, daß man N-Acetylglucosamin zur erwähnten Therapie verwendet.

Bevorzugte Ausführungsformen sind in den Unteransprüchen angegeben.

Es konnte überraschend festgestellt werden, daß sich Glucosamin durch N-Acetylglucosamin ersetzen läßt. Beide Substanzen müssen, um in die GAGs eingebaut zu werden, zuerst phosphoryliert werden. Bisher wurde angenommen, daß N-Acetylglucosamin als Vorstufe für die Synthese von Proteoglycanen im Säugerorganismus keine oder allenfalls eine unbedeutende Rolle spiele, vgl. Vidal y Plana, R.R. und K. Karzel: Glukosamin; Seine Bedeutung für den Knorpelstoffwechsel der Gelenke; 1. Biochemie der Proteoglykane, Untersuchungen an in-vitro Kulturen embryonaler Mäuse-Fibroblasten und Knochenanlagen. Fortschr. Med. 98, 55-594, 1980.

N-Acetylglucosamin bietet gegenüber Glucosamin und dessen Salzen, z.B. dem Sulfat, bedeutende Vorteile:

a) Wesentlich bessere Stabilität in wässriger Lösung.

b) Wesentlich geringere Osmolatität der äquimolaren Lösung wegen des fehlenden ionischen Charakters (kein Anion zur Neutralisation) und dem Fehlen eines Puffers zur Anpassung des pH-Wertes.

c) Keine Notwendigkeit des Zusatzes eines Lokalanästhetikums.

d) Eindeutig bessere Verträglichkeit bei parenteraler Anwendung.

e) Mindestens gleicher, offensichtlich sogar stärkerer Einbau in die Glucosaminoglycane der Gelenke nach Verabreichung von N-Acetylglucosamin als nach Verabreichung von Glucosamin-Salzen. Vergleichswerte sind in Tabelle 1 zusammengestellt.

Zusätzliche Versuche haben inzwischen bestätigt, daß N-Acetylglucosamin, insbesondere nach der für die Therapie erwünschten oralen Verabreichung, in mindestens dreifach höherer Menge als Glucosamin in die Gelenkknorpel eingebaut wird.

Die Erfindung betrifft ferner die Verwendung von N-Acetylglucosamin zur Herstellung von Arzneimitteln für die Therapie degenerativer Erkrankungen der Gelenke und des Binde-und Stützgewebes sowie verwandter Erkrankungen.

N-Acetylglucosamin kann allein oder in Kombination mit gebräuchlichen Hilfsstoffen als Lösung für die intraartikuläre, intravenöse, intramuskuläre oder sonstige Injektion oder Infusion oder auch für die orale Einnahme zubereitet werden. Für die orale Einnahme ist N-Acetylglucosamin nicht zuletzt wegen des angenehmen Geschmacks auch als Pulver oder Granulat geeignet. Es kann ebenso für die exakte Dosierung mit oder ohne pharmazeutische Hilfsstoffe in Kapseln gefüllt oder als Tabletten bzw. Dragees zubereitet werden. Da N-Acetylglucosamin chemisch stabil und relativ inert ist, lassen sich Kombinationspräparate mit vielen anderen Arzneistoffen herstellen, ohne daß es zu einem Wirkungsverlust eines der Bestandteile kommt.

Für die Injektion oder Infusion sind wässrige Lösungen von N-Acetylglucosamin mit einer Konzentration von 5-500 mg/ml und Volumina von etwa 0,5 -1000 ml geeignet. Gegebenenfalls muß die Osmolalität der Lösungen (unter ca. 60 mg N-Acetylglucosamin/ml) durch Zusatz von beispielsweise NaCl oder Glucose dem physiologischen osmotischen Druck angepaßt werden. Die Gesamtdosis liegt vorzugsweise zwischen 100 und 5000 mg N-Acetylglucosamin. Bei der oralen Verabreichung liegt die bevorzugte Dosierung zwischen 500 mg und 5000 mg/Tag.

Durch die Verwendung von N-Acetylglucosamin ist es erstmals möglich, die für eine wirksame Arthrose-Therapie notwendige Injektionsform als gut verträgliches, über lange Zeit stabiles und unkompliziert anwendbares Präparat ohne unerwünschte Zusätze anzubieten. N-Acetylglucosamin ist besser verträglich als Glucosaminsulfat und wird überraschenderweise auch zu einem höheren Anteil in die Glycosaminoglycane der Gelenke eingebaut.

## TABELLE 1

Einbau von Glucosamin und N-Acetylglucosamin in die Glycosaminoglycane der Gelenke der Maus.

Etwa $10\mu$Ci N-Acetyl-$^{14}$C-Glucosamin oder $^{3}$H-Glucosamin wurden Mäusen entweder intramuskulär oder dreimal im Abstand von sechs Stunden oral verabreicht. Etwa 24 Stunden nach der (letzten) Applikation wurden die Tiere getötet und der Einbau der markierten Substanzen in die GAGs der Gelenke der hinteren Extremitäten bestimmt.

| Substanz | Applikations-weise | Dosis ($\mu$Mol) | nMol in den GAGs einer Extremität |
|---|---|---|---|
| Glucosamin | 3 x p.o. | 1,7 | 0,013 |
| | | | 0,0099 |
| | | | 0,018 |
| | | | 0,019 |
| N-Acetyl-glucosamin | 3 x p.o. | 1,8 | 0,039 |
| | | | 0,041 |
| | | | 0,047 |
| | | | 0,047 |
| Glucosamin | i.m. | 0,88 | 0,033 |
| | | | 0,013 |
| | | | 0,089 |
| | | | 0,052 |
| N-Acetyl-glucosamin | i.m. | 0,85 | 0,12 |
| | | | 0,094 |
| | | | 0,22 |
| | | | 0,21 |

## Beispiele

1. Lösung für die intravenöse, intramuskuläre oder intraartikuläre Injektion

400 g N-Acetylglucosamin p.a. werden in 3000 ml Aqua pro injectione gelöst; die Lösung wird steril filtriert und in 3 ml-Portionen in Ampullen gefüllt. Die Ampullen werden zugeschmolzen und 20 Min. bei 121°C sterilisiert. Der Wirkstoffgehalt liegt bei > 98%, der pH der gänzlich ungepufferten Lösung beträgt 6,3.

2. Lösung für die Infusion

6000 g N-Acetylglucosamin und 1800 g NaCl p.a. werden in 300 Liter Aqua pro injectione gelöst; die Lösung wird steril filtriert und in 50 ml Infusionsflaschen gefüllt. Die Flaschen werden 20 Min. bei 121°C autoklaviert.

3. Tabletten

2500 g N-Acetylglucosamin werden mit 1000 g Tablettose und 15 g Magnesiumstearat homogen vermischt, granuliert und zu Tabletten verpreßt.

Einzelgewicht einer Tablette: 351,5 mg

Gehalt an N-Acetylglucosamin je Tablette: 250 mg.

**Ansprüche**

1. Verwendung von N-Acetylglucosamin zur Therapie degenerativer Erkrankungen der Gelenke und des Binde-und Stützgewebes sowie verwandter Erkrankungen.

2. Verwendung von N-Acetylglucosamin nach Anspruch 1, **dadurch gekennzeichnet**, daß N-Acetylglucosamin als wässrige Lösung zur intraartikulären, intravenösen, intramuskulären oder sonstigen Injektion oder Infusion oder oralen Einnahme eingesetzt wird.

3. Verwendung von N-Acetylglucosamin nach Anspruch 1, **dadurch gekennzeichnet**, daß N-Acetylglucosamin in fester Form zur Herstellung von Pulvern, Granulaten, Kapseln, Tabletten oder Dragees eingesetzt wird.

4. Verwendung von N-Acetylglucosamin nach mindestens einem der Ansprüche 1 bis 3, **dadurch gekennzeichnet**, daß N-Acetylglucosamin unter Zusatz unbedenklicher und üblicher pharmazeutischer Trägerund Hilfsstoffe zur Anpassung der Osmolalität, Pufferung, Stabilisierung, Formgebung und Steuerung der Freigabe und Bioverfügbarkeit eingesetzt wird.

5. Verwendung von N-Acetylglucosamin zur Herstellung eines Arzneimittels für die Therapie degenerativer Erkrankungen der Gelenke und des Binde-und Stützgewebes sowie verwandter Erkrankungen.

6. Verwendung von N-Acetylglucosamin zur Herstellung eines Arzneimittels nach Anspruch 5, **dadurch gekennzeichnet**, daß das N-Acetylglucosamin in fester Form oder als wässrige Lösung zusammen mit pharmazeutisch unbedenklichen Träger-und/oder Hilfsstoffen eingesetzt wird.